# EUROPEAN PATENT APPLICATION

(11) **EP 4 258 281 A1**
(43) Date of publication of application: **11.10.2023**
(21) Application number: 22167382.5
(22) Date of filing: 08.04.2022
(51) Int. Cl.: G16H 50/30

(54) **METHOD FOR STRESS REDUCTION**

(71) Applicant: GlaxoSmithKline Consumer Healthcare Holdings (US) LLC, Wilmington, DE 19808 (US)
(72) Inventor: Crosier, Dario, Weybridge, KT13 0DE (GB); Echiadis, Angelos, Weybridge, KT13 0DE (GB); Babaian, Nicholas, Weybridge, KT13 0DE (GB)
(74) Representative: Gundel, Isabelle

(57) **Abstract**

The present invention relates to a computer implemented method for providing recommendations for managing stress to a user wearing a device comprising a physiological sensor, comprising the following steps:
a. Receiving physiological data from the physiological sensor,
b. Determining at least one personal health index at regular intervals based on the physiological data,
c. Monitoring an evolution over time of the health index,
d. Determining a recommendation on the intake of a nutritional supplement according to the evolution over time of the personal health index,
e. displaying the recommendation to the user.

The invention further relates to a kit comprising a nutritional supplement and an app which is designed to carry out such a method.

## Description

### FIELD OF THE INVENTION

This invention relates to a computer implemented method for supporting a user through stress and improving the mental resilience of the user. The invention further relates to a health and wellbeing improvement kit which includes a nutritional supplement and a computer programme product adapted to perform such a method.

### BACKGROUND TO THE INVENTION

Stress can be defined as the brain-body reaction of being overwhelmed or unable to cope with mental or emotional pressure arising as a result of internal or external stimuli. Anxiety commonly occurs concomitantly as the body's natural response to stress. Anxiety may cause feelings of panic, fear and tension. The organisation of the body's response to stress involves neurotransmitter systems and neuronal responses to hormones and chemicals in several areas of the limbic system. Stress and anxiety related disorders in humans have a significant impact globally. In 2020, an estimated 40% of adults worldwide experienced "a lot of stress the previous day" a 5% increase from 2019 (Gallup World Poll Data Set, 2020). Further, the monetary impact of work-related stress alone has been estimated to cost the U.S. economy $402 billion (European Agency for Safety and Health at Work, 2014). This increasing occurrence of stress and anxiety is cause for a growing concern on the impact to healthcare systems. It highlights a need for accessible stress and anxiety management tools for individuals to build resilience to reduce the global economic burden of stress related disorders.

To regulate an acute stress response to an external stimulus, neurotransmitters including dopamine, acetylcholine, glutamate and γ-aminobutyric acid (GABA) are released. In the case of chronic stress, the parts of the limbic system controlled by the GABAergic system may be impaired. There is evidence to suggest that levels of GABA activity, one of the primary neurotransmitters in the brain, may be altered in stress related disorders and that this may contribute in some way to the pathogenesis of stress-related diseases. Specifically, there are regions of the brain where reduction in GABA activity has been demonstrated in stress related disorders, including areas of the cortex such as the Insula and anterior cingulate.

All stress responses to internal or external stimuli are individual, and depending on how an individual adapts and develops mechanisms of stress resilience, there will be physiological and behavioural differences. As a result, the therapeutic management of stress aiming at an increased stress resilience and ultimately increased health and wellbeing requires an individualised approach and ongoing monitoring and adjustment.

Therapeutic management to build resilience for stress and anxiety encompasses both pharmacological and non-pharmacological interventions. Diverse therapeutic management approaches from Western, Indian, Chinese and other cultures are increasingly combined to create individualised, holistic health and wellbeing solutions for stress resilience. Stress and anxiety management tools may include, *inter alia,* the use of nutritional supplements, cognitive behavioural therapy (CBT), meditation, diaphragmatic breathing, mindfulness, visualisation and imagery and the building and activation of social support networks, but also pharmacological interventions based on antidepressants or other drugs.

Among the non-pharmacological interventions, behavioural change recommendations are important. The most extensively researched form is cognitive behavioural therapy (CBT). CBT focusses on modifying thinking and transferring behavioural skills learnt in talk therapy with a qualified therapy to everyday life. As CBT treatment continues, the patient can transition from relying on their therapist to becoming their own therapist through self-management of these skills. With the advancement of Artificial Intelligence (AI), CBT can be delivered with computer implemented methods to tailor individual treatment.

The use of nutritional supplements is another stress and anxiety management tool. In particular, certain probiotic bacterial strain supplements, known as psychobiotics, have been clinically evidenced to have a positive effect on emotional attention and stress resilience. Computer-implemented methods using physiological data provided by a wearable and aiming at recognizing or tracking emotions of a user have been described for example in US2018/014739A1 and US2020/0074039A1.

With millions of undiagnosed individuals suffering from either acute or chronic stress and anxiety there is an unmet and growing need for accessible and individualised solutions to this global problem.

It is thus an objective of the present invention to provide a solution for individualised stress management.

### SUMMARY OF THE INVENTION

According to a first aspect of the invention, a computer implemented method is provided for providing recommendations for managing stress to a user wearing a device comprising a physiological sensor.

According to the invention, the method comprises the following steps:
a. Receiving physiological data from the physiological sensor,
b. Determining at least one personal health index at regular intervals based on the physiological data,
c. Monitoring an evolution over time of the health index,
d. Determining a recommendation on the intake of a nutritional supplement according to the evolution over time of the personal health index,
e. displaying the recommendation to the user.

According to a preferred embodiment of the invention, the physiological sensor is a heart rate sensor, and/ or a galvanic sensor and/ or an accelerometer.

According to another preferred embodiment of the invention, the physiological data used in step b) comprises data selected from heart rate variability data, electro-dermal activity data, respiration rate data and physical activity data.

Preferably, the method comprises a further step of receiving self-reported data and/or mobile phone usage data, wherein the self-reported data and/or mobile phone usage data is used in step b) to determine the personal health index. The self-reported data can relate for example to data relating to intake of the nutritional supplement, to other nutrition information, data on emotions which the user experiences, or data relating to a health state. The mobile phone usage data can for example be activity data, sleep data, or data relating to usage of social media, messaging apps or media consumption.

According to a preferred embodiment of the invention, the health index determined in step b) is a stress index, and in a further step b)', a second personal health index, herein also referred to as a resilience index, is determined based on the stress index and its evolution over time.

According to an embodiment of the invention, the nutritional supplement has a composition comprising at least one bacterial strain of the species Lactobacillus helveticus, Bifidobacterium longum and/or Lactobacillus plantarum. Bifidobacteria in the gut produce neurotransmitters through metabolism of indigestible fibres. GABA and other neurotransmitters produced by bacteria in the gut may in some cases cross the blood brain barrier and alter brain activity.

These particular bacterial strains are clinically evidenced to have a positive effect on mental health by acting on the gut-brain axis and by increasing GABA activity.

According to a preferred embodiment of the invention, the nutritional supplement has a composition comprising Lactobacillus helveticus Rosell- 52 (CNCM-I-1722), Bifidobacterium longum Rosell-175 (CNCM-I-3470) and/or Lactobacillus plantarum Rosell-1012 (CNCM-I-3736). This combination of three psychobiotics has been shown to alter functional activity in the insula, as well as in lateral orbital and ventral cingulate gyri, in a proof-of-concept study utilizing functional MRI, and it seems likely that the combination of the specific strains has a synergistic effect on positive emotional regulation of brain activity in regions related to stress and anxiety.

According to another preferred embodiment of the invention, the nutritional supplement has a composition comprising an antioxidant such as L-glutathione, and/or minerals such as potassium, magnesium, and zinc, and/or dietary fibers such as inuline, in addition to at least one bacterial strain of the species Lactobacillus helveticus, Bifidobacterium longum and/or Lactobacillus plantarum. preferably in addition to Lactobacillus helveticus Rosell- 52 (CNCM-I-1722), Bifidobacterium longum Rosell-175 (CNCM-I-3470) and/or Lactobacillus plantarum Rosell-1012 (CNCM-I-3736). Minerals such as potassium, magnesium and zinc have additional anti-stress benefits.

According to another preferred embodiment of the invention, the nutritional supplement has a composition comprising L-theanine and/or Ashwagandha, preferably in addition to at least one bacterial strain of the species Lactobacillus helveticus, Bifidobacterium longum and/or Lactobacillus plantarum. The inclusion of L-theanine or Ashwagandha is advantageous because these ingredients, known as adaptogens, have been shown to reduce user perception and physiological biomarkers of stress. The inclusion of these adaptogens in the supplement will therefore have a complementary effect on the efficacy by providing stress-protective effects.

Generally, it is advantageous if the nutritional supplement has a composition shown to alter functional activity in brain regions implicated in emotional and cognitive processing comprising the insula, lateral orbital and ventral cingulate gyri.

According to an embodiment of the invention, step c) comprises comparing the personal health index to a threshold, and step d) comprises a recommendation to alter the nutritional supplement intake when the personal health index exceeds the threshold for a pre-determined amount of time.

The threshold can be a predetermined percentage of an average value for the particular user or for a specific group of users, or it can be adjusted based on feedback from the user.

According to an embodiment of the invention, the recommendation to alter the nutritional intake recommendation comprises a recommendation to change the supplement formulation and/or to increase the dosage, and/or to change the time of day on which the supplement is taken.

According to a preferred embodiment of the invention, the method comprises an additional step d') of determining a behavioural change recommendation, and an additional step e') of displaying said behavioural change recommendation to the user. The combination of a suitable behavioural change recommendation with the nutritional supplement can produce an additive effect on GABAergic activity to build stress resilience. Behavioural change recommendations can for example comprise the recommendation to consult specific educational material, to follow guided exercises or to follow CBT coaching delivered through calls or messages.

Another aspect of the invention is a health and wellbeing improvement kit comprising a nutritional supplement and a computer programme product adapted to perform the steps of the method described above.

According to a preferred embodiment, the nutritional supplement is a probiotic composition for oral administration. More preferably, it has a composition comprising at least one bacterial strain of the species Lactobacillus helveticus, Bifidobacterium longum and/or Lactobacillus plantarum.

According to an even more preferred embodiment, the nutritional supplement has a composition comprising Lactobacillus helveticus Rosell- 52 (CNCM-I-1722), Bifidobacterium longum Rosell-175 (CNCM-I-3470) and/or Lactobacillus plantarum Rosell-1012 (CNCM-I-3736).

According to a preferred embodiment, the nutritional supplement is a probiotic composition for oral administration comprising (a) probiotics in an amount of at least about: *Bifidobacterium longum* 50 billion CFU/g, *Lactobacillus helveticus* 150 billion CFU/g, and *Lactobacillus plantarum* 150 billion CFU/g, (b) one or more prebiotic components selected from inulin, fructose, fructooligosaccharides and mixtures thereof, and (c) one or more metal salts selected from magnesium gluconate, potassium citrate, zinc gluconate, and copper citrate.

According to a preferred embodiment of the invention, the supplement further comprises glutathione, for example L-glutathione. Glutathione is an anti-oxidant which can fight oxidative stress.

According to preferred embodiment, the nutritional supplement which is part of the kit according to the invention further comprises L-glutathione, Potassium, Inulin, Magnesium, and Zinc, preferably in combination with L-theanine and/or Ashwagandha.

According to a preferred embodiment, the nutritional supplement is a probiotic composition for oral administration comprising:
at least about 50 billion CFU/g *Bifidobacterium longum;*
at least about 150 billion CFU/g *Lactobacillus helveticus,*
at least about 150 billion CFU/g *Lactobacillus plantarum;*
a prebiotic component;
a mineral metal salt; and
an excipient.

Preferably, *Bifidobacterium longum* is *Bifidobacterium longum* R175.

Preferanbly, *Lactobacillus helveticus* is *Lactobacillus helveticus* R52.

Preferably, *Lactobacillus plantarum* is *Lactobacillus plantarum* R1012.

The one or more prebiotic components are preferably selected from a group consisting of inulin, fructose, fructooligosaccharides, and any combination thereof.

The one or more metal salts are preferably selected from a group consisting of magnesium, potassium, copper, zinc, and any combination thereof.

Preferably, the magnesium salt is magnesium gluconate, the potassium salt is potassium citrate, the copper salt is copper citrate, and the zinc is zinc gluconate.

Another aspect of the invention is a computer implemented method for detection and initial diagnosis of clinically relevant anxiety where an individual would benefit from a consultation with a healthcare professional, according to the criteria set out in the Diagnostic and Statistical Manual of Medal Disorders (DSM-5), published by the American Psychiatric Association. The user wears or carries a device comprising a physiological sensor, and the method comprises the following steps:
a. Receiving physiological data from the physiological sensor,
b. Determining at least one personal health index at regular intervals based on the physiological data,
c. Monitoring an evolution over time of the health index,
d. Determining a recommendation on the intake of a nutritional supplement according to the evolution over time of the personal health index,
e. displaying the recommendation to the user.

### Physiological Data

Detection of momentaneous stress or anxiety is possible in real-life conditions using commercially available wearable devices. Signals related to stress detection which are measured by existing wearable devices include:
- Heart Rate (HR) - extracted from Electrocardiography (ECG) or Photoplethysmography (PPG), the latter being the preferred measurement modality in consumer-grade wearable devices. PPG sensors are available across most wearable devices currently on the market.
- Heart Rate Variability (HRV) - extracted from ECG or PPG, it is associated with the Autonomic Nervous System (ANS) and can provide information on the activation of the Sympathetic branch of ANS under stress. Most high-grade wearable devices report HRV as a standard parameter.
- Electro-Dermal Activity (EDA) - extracted from a Galvanic Skin Response (GSR) sensor measuring skin conductance, which is known to vary as a result of sweat gland activity during stress-induced ANS activation. EDA is currently mostly present in medical-grade wearable devices, and GSR sensors are rarely included in wearable devices targeting a broad audience. GSR is known as being linked to stress and arousal.
- Respiration Rate (RR) - extracted from ECG or PPG, it can be associated with increased cardio-respiratory activity as a result of stress. Respiration rate is currently not considered to be a mainstream parameter for consumer-grade wearables, namely due to technical difficulties in the correct extraction.
- Acceleration (ACC) - extracted from accelerometers and gyroscopes to measure behaviour patterns and activity levels, considered to be a standard parameter across all wearable devices. Secondary parameters of relevance to stress which are predominantly calculated from accelerometer signals are sleep and activity.

It should be understood that any combination of these parameters has the potential to detect and possibly quantify the degree of sympathetic ANS activation, and can thus to serve as the basis to determine a relevant personal health index relating to stress and/or anxiety.

### Determination of a personal health index

In the context of the present patent application, a personal health index corresponds to a value expressed on a suitable scale, for example from 0 to 100%, derived from an algorithm that uses one or several physiological parameters, namely those mentioned above (HR, HRV, EDA etc.). Optionally, such an algorithm can also use self-reported parameters (e.g. perceived stress levels, objective stress events etc.) and /or engagement/usage metrics related to CBT or journaling activities as well as to various de-stressing exercises (e.g. deep breathing, mindfulness etc.).

A first personal health index, also referred to as stress index, can be determined based on physiological data which is acquired by physiological sensors which can be integrated in wearable device such as a wristband, a watch, or similar. Alternatively or in addition, the physiological data can be acquired by physiological sensors, for example accelerometers, which are integrated in a smartphone, or into other non-wearable devices which communicate with a smartphone, such as connected bathroom scale, toothbrush, transcutaneous electric nerve stimulation (TENS) device or the like. Alternatively or in addition, physiological data can be self-reported data, such as data body weight, sleep quality and quantity, or data relating to physical stress symptoms experienced by a user. Data relating to nutritional intake and data relating to intake of nutritional supplements, namely the type of supplements used, the amount, and time of intake can also be used. Based on all or some of the afore-mentioned the data, the algorithm will determine a first personal health index or stress index, which will indicate what might be the level of stress in the moment. A typical response to a stress event might be a decrease in heart rate variability, a change in behaviour, an increased heart rate, or a combination of these in relation to their respective previous baseline values. I such a case the stress index would increase, indicating that stress in this moment is higher than stress a few moments ago.

The stress index may be a relative value that is baselined every day, or it may use average values of the relevant physiological parameters over a longer period of time as a baseline. In either case, the purpose of the stress index is mainly to provide an indication of "in-the-moment" stress severity, rather than indicating the average level of stress over several days and weeks.

The stress index can also be used to give feedback to the user, and prompt him or her to engage with the app for journaling when the stress index exceeds a pre-determined threshold.

In addition to the first personal health index, also referred to as stress index, the method according to the invention may use a second personal health index, also referred to as a resilience index. The purpose of the resilience index is to provide information on the more mid- and long-term stress levels both in terms of severity and duration, and on long-term progress of resilience against stress, using a different combination of parameters.

The same type of physiological data already described above could be used, as well as corresponding derivatives, namely before and after stress events, i.e. before and after peaks of the stress index, or before and after the intake of a nutritional supplement, before and after a CBT session or a workout, etc. Peak and differential values of certain parameters may indicate stress severity, while roll-off rates (delta-over-time) could indicate recovery speed. A small response of the former and/or a fast roll-off of the latter are both associated with increased resilience, which results in a higher score.

In additional to physiological data, self-reported data can be used to calculate the resilience index. Such self-reported data can relate to perceived stress, but it can also comprise information relating to specific keywords and positive/negative emotions captured in journaling. The algorithm can for example use parameters such as a frequency of stress events, perceived stress scores, frequency of using negative keywords- The use of specific keywords in journaling for example might be a clear indicator for recession to a lower resilience level, or on the other hand, the use of certain positive, predetermined keywords may indicate a rising resilience index. Furthermore, additional metrics can be calculated such as "stress-free minutes" similarly to "exercise minutes" used in smartwatches, and can be shared with a user to allow them to see whether their resilience has increased and/or whether their stress levels have decreased.

### Additional app-derived data

The method according to the invention can be implemented in an app which users can use on a smartphone, a tablet, a computer, or any other suitable device. Additional, non-physiological data derived from the app can be used to detect specific patterns that can be indicative of changing resilience to stress. The App can prompt the user to journal on his emotions and the underlying causes when a peak in heart rate and/or heart rate variability is detected, which cannot be explained by physical exercise. Users are prompted to describe what they feel, by what the feeling was caused, etc. The app can use machine learning to continuously improve the capability to detect emotions based on the user entries and the corresponding physical data.

Usage metrics like "minutes of engagement" with the app; e.g. logging of time and frequency of journaling, coaching, breathing, mindfulness or other types of engagements could indicate shifts in behaviour patterns that may be indicative of a changing resilience. For example, a downtrend of breathing exercise frequency combined with a low stress index may indicate incremental resilience to stress. Additionally, other types of objective data derived from the app could provide insights on the current state of mood while carrying out journaling; such as speed of typing. For example, a low speed might indicate a state of calmness, while the change of typing speed during a journaling session could indicate the effectiveness of that session, as well provide information on the impact of the stressor associated to the same session. In general; a plurality of app usage parameters could be used to provide not only context to the journaling session (e.g. feeling angry or sad), but it could also reveal other information that would otherwise be evident in a face-to-face discussion. E.g. going back and forth editing and re-writing sections of the journal would indicate uncertainty, which could be a trigger for a follow-up discussion with a coach.

### Intake of a nutritional supplement

It is known that probiotics can have not only physiological effects, namely due to them altering the gut microbiome, but also an effect of a user's resilience to stress, on sleep quality, and on mental wellbeing overall. These effects depend on parameters such the users' initial level of stress and gender, on the microbiome present, and other factors, which are not all well understood.

The efficacy of a supplement containing probiotics can also vary depending on the conditions of consumption, and namely whether they are taken with a meal or not. At least certain uncoated probiotics are known to survive best when taken with fat, while others have higher survival rates when taken on an empty stomach.

The diversity of the microbiome is an important factor for physical and mental wellbeing, and a rotation between different strains and types of probiotics has therefore been found to be more advantageous than a long-term daily consumption of the same probiotic supplement, together with a diet which is equally constant over time.

### DETAILED DESCRIPTION OF THE INVENTION

### EXAMPLES

In an embodiment, when buying a probiotic nutritional supplement, which is part of a range of nutritional supplement products, users will be prompted via a QR code on pack or in a product leaflet, to download an app which will help them to better manage their stress, and adapt the intake of the nutritional supplement to their needs. At least one product in the range will comprise Lactobacillus helveticus Rosell- 52 (CNCM-I-1722), Bifidobacterium longum Rosell-175 (CNCM-I-3470), Lactobacillus plantarum Rosell-1012 (CNCM-I-3736), L-glutathione, Potassium, Inulin, Magnesium, and Zinc.

Alternatively, users can as a first step download an app, through which the nutritional supplement can be ordered. The app can include a subscription service including a certain amount of nutritional supplements per month.

The app contains a software which calculates a first personal health index, also called stress index, between 0% and 100%, using heart rate, heart rate variability, acceleration data, and optionally skin temperature and galvanic skin response. Heart rate and heart rate variability are measured using photoplethysmography (PPG) sensor integrated into a smartwatch, or into a wearable device such as a wristband. Skin temperature is measured using a thermometer integrated into a smartwatch or a wearable. Galvanic skin response is measured using an appropriate sensor integrated into a wearable device or a smart watch. If the user's wearable device or smartwatch do not have temperature and/or galvanic skin response sensors, the algorithm will use only the other available data.

It should be noted that the actual calculation can also be carried out on a remote server, with the app itself only sending the necessary data to the server.

Acceleration data can be provided either by an accelerometer integrated in the smartwatch, or by an accelerometer or gyroscope integrated in a mobile phone. Typically, a higher heart rate which is not due to physical exercise and a lower heart rate variability will be indicative of a higher stress level, and will therefore lead to an increase in the stress index. As a baseline, average values over the last 7 to 28 days, if available, will be used. When users first used the app, and average value for a similar group of users (e.g. users having the same gender and age, for example) can be used.

A second personal health index, also called resilience index, between 0% and 100% is calculated, using the derivative of the stress index, i.e. its evolution over time. Furthermore, users will also be asked to enter data relating to their feelings, activities, and perceived stress levels into an app (socalled journaling). This data will also be used to determine the resilience index. Typically, a lower variability and a lower average of the stress index over time will correspond to a higher resilience index, i.e. the user copes better with stress. Equally, certain keywords used in the journaling activity, indicative of a relaxed, positive state of mind, will lead to an increase in the resilience index, whereas other keywords corresponding to a stressed, negative state of mind, will lead to a decrease in the resilience index. Metadata such a information on the speed of typing, the length of the journal entries, whether the user edits the entries or not is also used.

The user will also enter daily information relating to the intake of a nutritional supplement in the app, namely whether any supplements are taken, and if so, which product, in which amount, and at what time of the day.

Based on all of the above data, a recommendation relating to the intake of a nutritional supplement is determined and displayed to the user.

In particular, if the resilience index is increasing, the recommendation may be to not modify the intake of the nutritional supplement, or to lower the does.

If the resilience index is decreasing, or at least not increasing, then a recommendation to alter the intake of the nutritional supplement will be given.

The app may also offer the possibility to track whether the user has actually taken the nutritional supplement according to the recommendation, either via input given by the user, or via technical means. If the app determines that the user has not or not regularly taken the recommended supplement, it may display additional reminders and/or recommendations to take the supplement as per the current recommendation, in particular if the resilience index is decreasing or not increasing.

The app will also automatically set a corresponding reminder, which will be displayed to the user via a push-message or similar to remind the user when a supplement should be taken. This reminder can include information on the kind of supplement, and the recommended amount. The app can also remind the user to re-order or purchase appropriate nutritional supplements, and suggest automatic re-ordering. If the app offers a subscription, an appropriate supplement could also automatically be sent to a user.

The recommendation may comprise a recommendation to increase the daily dose of the nutritional supplement, and/or to increase the frequency of intake.

The recommendation may comprise a recommendation to switch from a supplement containing only probiotics, to a supplement containing additionally also adaptogens such as L-theanine or Ashwagandha. Of course, the different ingredients may also be present in separate products, and the recommendation could thus also be to take an additional supplement containing a complementary ingredient.

Depending on the type of nutritional supplement, and as a function of available information on whether this specific supplement has best effects when taken with a meal, specifically with fats or, or whether this specific supplement has best effects when taken on an empty stomach, the recommendation may comprise a recommendation to modify the timing of the intake of the nutritional supplement (i.e. with a meal rather than before going to bed, in the morning before the first meal, etc.).

If the available data suggests that the same type of probiotic supplement has been taken for a certain period of time already, for example for more than 2 weeks, the recommendation may comprise a recommendation to switch to another probiotic supplement containing different bacteria or different strains of the same bacteria, to increase the diversity of the user's microbiome.

If the stress index and the resilience index indicate a series of extreme stress events and a poor recovery rate, the recommendation may comprise a recommendation to increase the intake of magnesium to compensate for a loss of magnesium through stress.

If the stress index is increasing and/or the resilience index is decreasing, and sleep quality derived from accelerometer data sleep is also poor, the recommendation can comprise a recommendation to take a nutritional supplement containing probiotics and / or adaptogens such as L-theanine or Ashwagandha with dinner.

In addition to recommendations relating to the intake of a nutritional supplement, a recommendation to adopt certain behaviours can be determined and displayed. As an example, a recommendation may be to use a CBT functionality of the App, to sign-up for coaching sessions, or to consult a health care professional.

## Claims

1. A computer implemented method for providing recommendations for managing stress to a user wearing or carrying a device comprising a physiological sensor, comprising the following steps:
a. Receiving physiological data from the physiological sensor,
b. Determining at least one personal health index at regular intervals based on the physiological data,
c. Monitoring an evolution over time of the health index,
d. Determining a recommendation on the intake of a nutritional supplement according to the evolution over time of the personal health index,
e. displaying the recommendation to the user.

2. A computer implemented method according to claim 1, wherein the physiological sensor is a heart rate sensor, and/ or a galvanic sensor and/ or an accelerometer.

3. A computer implemented method according to claim 1 or 2, wherein the physiological data used in step b) comprises data selected from heart rate variability data, electro-dermal activity data, respiration rate data and physical activity data.

4. A computer implemented method according to any of the preceding claims, comprising a further step of receiving self-reported data and/or mobile phone usage data, wherein the self-reported data and/or mobile phone usage data is used in step b) to determine the personal health index.

5. A computer implemented method according any of the preceding claims, wherein the health index determined in step b) is a stress index, and in a further step b)', a second personal health index is determined based on the stress index and its evolution over time.

6. A computer implemented method according to any of the preceding claims, wherein the nutritional supplement has a composition comprising at least one bacterial strain of the species Lactobacillus helveticus, Bifidobacterium longum and/or Lactobacillus plantarum.

7. A computer implemented method according to any of the preceding claims, wherein the nutritional supplement has a composition comprising Lactobacillus helveticus Rosell-52 (CNCM-I-1722), Bifidobacterium longum Rosell-175 (CNCM-I-3470) and/or Lactobacillus plantarum Rosell-1012 (CNCM-I-3736).

8. A computer implemented method according to claim 7, wherein the nutritional supplement has a composition further comprising L-glutathione, Potassium, Inulin, Magnesium, and Zinc.

9. A computer implemented method according to any of claims 6 to 8, wherein the nutritional supplement has a composition further comprising L-theanine and/or Ashwagandha.

10. A computer implemented method according to any of the preceding claims, wherein step c) comprises comparing the personal health index to a threshold, and step d) comprises a recommendation to alter the nutritional supplement intake when the personal health index exceeds the threshold for a pre-determined amount of time.

11. A computer implemented method according to claim 10, wherein the recommendation to alter the nutritional intake recommendation comprises a recommendation to change the supplement formulation and/or to increase the dosage, and/or to change the time of day on which the supplement is taken.

12. A computer implemented method according to claim 10 or 11, wherein the recommendation to alter the nutritional intake recommendation comprises a recommendation to change the supplement formulation from a formulation containing certain probioticis, to a formulation containing other probiotics, or different strains of the same probiotics.

13. A computer implemented method according to any of the preceding claims, comprising an additional step d') of determining a behavioural change recommendation, and an additional step e) of displaying said behavioural change recommendation to the user.

14. A health and wellbeing improvement kit comprising a nutritional supplement and a computer programme product adapted to perform the steps of the method of any of the preceding claims.

15. A health and wellbeing improvement kit according to claim 13, wherein the nutritional supplement has a composition comprising at least one bacterial strain of the species Lactobacillus helveticus, Bifidobacterium longum and/or Lactobacillus plantarum.

16. A health and wellbeing improvement kit according to claim 13 or 14, wherein the nutritional supplement has a composition comprising Lactobacillus helveticus Rosell- 52 (CNCM-I-1722), Bifidobacterium longum Rosell-175 (CNCM-I-3470) and/or Lactobacillus plantarum Rosell-1012 (CNCM-I-3736).

17. A health and wellbeing improvement kit according to claim 15 or 16, wherein the nutritional supplement further comprises an anti-oxidant, namely L-glutathione, a pre-biotic, and a metal salt.

18. A health and wellbeing improvement kit according to any of claims 13 to 16, wherein the nutritional supplement has a composition further comprising L-theanine and/or Ashwagandha.

19. A computer implemented method detection and initial diagnosis of clinically relevant anxiety where an individual would benefit from a consultation with a healthcare professional to a user wearing a device comprising a physiological sensor, comprising the following steps:
f. Receiving physiological data from the physiological sensor,
g. Determining at least one personal health index at regular intervals based on the physiological data,
h. Monitoring an evolution over time of the health index,
i. Determining a recommendation on the intake of a nutritional supplement according to the evolution over time of the personal health index,
j. displaying the recommendation to the user.
